(19) Europäisches Patentamt
European Patent Office
Office européen des brevets

(11) Veröffentlichungsnummer: **0 035 706**
**B1**

# (12) EUROPÄISCHE PATENTSCHRIFT

(45) Veröffentlichungstag der Patentschrift: **25.01.84**

(51) Int. Cl.³: **C 07 D 253/06**

(21) Anmeldenummer: **81101403.4**

(22) Anmeldetag: **26.02.81**

## (54) Verfahren zur Herstellung von 4-Amino-6-tert.-butyl-3-methylthio-1,2,4,-triazin-5(4H)-on.

(30) Priorität: **08.03.80 DE 3009043**

(43) Veröffentlichungstag der Anmeldung:
**16.09.81 Patentblatt 81/37**

(45) Bekanntmachung des Hinweises auf die Patenterteilung:
**25.01.84 Patentblatt 84/4**

(84) Benannte Vertragsstaaten:
**AT BE CH DE FR GB IT LI NL**

(56) Entgegenhaltungen:
**DE - A - 2 708 185**
**DE - A - 2 732 797**

**Die Akte enthält technische Angaben, die nach dem Eingang der Anmeldung eingereicht wurden und die nicht in dieser Patentschrift enthalten sind.**

(73) Patentinhaber: **BAYER AG, Zentralbereich Patente, Marken und Lizenzen, D-5090 Leverkusen 1, Bayerwerk (DE)**

(72) Erfinder: **Bonse, Gerhard, Dr., Wolfskaul 3, D-5000 Köln 80 (DE)**
Erfinder: **Blank, Heinz Ulrich, Dr., Am Geusfelde 35, D-5068 Odenthal (DE)**
Erfinder: **Krätzer, Hans, Dr., Am Acker 17, D-5600 Wuppertal 1 (DE)**

**EP 0 035 706 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Verfahren zur Herstellung von 4-Amino-6-tert.-butyl-3-methylthio-1,2,4-triazin-5-[4H]-on

Die vorliegende Erfindung betrifft ein von Pivaloylcyanid ausgehendes Verfahren zur Herstellung des bekannten, herbizid wirksamen 4-Amino-6-tert.-butyl-3-methylthio-1,2,4-triazin-5-[4H]-ons.

Es sind bereits Verfahren zur Herstellung von 4-Amino-6-tert.-butyl-3-methylthio-1,2,4-triazin-5-[4H]-on I, die von Pivaloylcyanid oder anderen Pivalinsäurederivaten ausgehen, bekanntgeworden. Sie unterscheiden sich in der Methode der Herstellung des Zwischenproduktes 4-Amino-6-tert.-butyl-3-mercapto-1,2,4-triazin-5-[4H]-on V.

Nach DE-OS Nr. 2165544 kann man das Triazinon I herstellen durch Umsetzung von Pivaloylchlorid mit einem Isonitril, Hydrolysieren des gebildeten Imidchlorids zum entsprechenden Trimethylbrenztraubensäureamid, Weiterumsetzung des Amids mit Thiocarbohydrazid und Methylierung des erhaltenen Cyclisierungsproduktes V (Ausbeuten: 60-82% d. Th. an V; 49-67% an I, jeweils bezogen auf Pivaloylchlorid).

Nach DE-OS Nr. 2221771 ist es möglich, das genannte Triazinon I herzustellen, indem man ein Pivalinsäureamid, z.B. Pivalanilid, durch Chlorierung, z.B. mittels Thionylchlorid, in das entsprechende Pivalimidochlorid überführt, dieses mit einem Metallcyanid, beispielsweise Kupfer(I)cyanid, oder Cyanwasserstoff gegebenenfalls in Gegenwart eines Katalysators zu dem entsprechenden α-Iminonitril umsetzt, letzteres durch Umsetzung mit Thiocarbohydrazid zu 4-Amino-6-tert.-butyl-5-imino-3-mercapto-1,2,4-triazin cyclisiert, dann die 5-Iminogruppe zur 5-Ketogruppe hydrolysiert, wobei man das Zwischenprodukt V erhält, und dieses anschliessend methyliert (Ausbeuten: 42-57% d. Th. an V; 35-47% d. Th. an I, jeweils bezogen auf Pivalanilid).

Beide Verfahren sind technisch ausserordentlich aufwendig, verlaufen mit unbefriedigenden Ausbeuten und sind damit zur Übertragung in den grosstechnischen Massstab ungeeignet.

Gemäss DE-OS Nr. 2733180 kann das genannte Triazinon I hergestellt werden, indem man Pivaloylcyanid in einer sogenannten Ritter-Reaktion mit t-Butanol oder Isobutylen zu Trimethylbrenztraubensäure-N-t-butylamide umsetzt und dieses, gegebenenfalls nach vorheriger Verseifung zur freien Trimethylbrenztraubensäure, mit Thiocarbohydrazid zu dem Zwischenprodukt V cyclisiert und letzteres anschliessend methyliert (Ausbeuten: 51-67% d. Th. an V; 41-54% an I, jeweils bezogen auf Pivaloylcyanid).

Das letztgenannte Verfahren hat den grundsätzlichen Nachteil, dass sich das als Zwischenprodukt auftretende Trimethylbrenztraubensäure-N-t-butylamid nur relativ schwer weiter umsetzen lässt; dies gilt sowohl für die Hydrolyse zur freien Ketosäure wie auch für die Ringschlussreaktion mit Thiocarbohydrazid.

So gelingt die saure Hydrolyse des Trimethylbrenztraubensäure-N-t-butylamids zu Trimethylbrenztraubensäure durch 10stündiges Erhitzen unter Rückfluss in 5N-HCl und nachfolgende extraktive Aufarbeitung mit $CH_2Cl_2$, verdünnter wässeriger NaOH-Lösung, konzentrierter Salzsäure und Essigester lediglich in einer Ausbeute von 75% d. Th.

Verzichtet man auf eine vorausgehende Hydrolyse des α-Ketocarbonsäure-N-t-butylamids und setzt dieses direkt mit Thiocarbohydrazid um, so kann das Cyclisierungsprodukt 4-Amino-6-t-butyl-3-mercapto-1,2,4-triazin-5-[4H]-on nach mehrstündigem Erhitzen (bis zu 8 h) unter Rückfluss lediglich in Ausbeuten von 72% d. Th. isoliert werden. Wie eigene Versuche darüber hinaus gezeigt haben, verläuft die Reaktion von α-Ketocarbonsäure-N-alkylamiden mit Thiocarbohydrazid nach DE-OS Nrn. 2165554 und 2733180 nicht einheitlich zu 4-Amino-6-t-butyl-3-mercapto-1,2,4-triazin-5-[4H]-on, sondern unter Bildung zahlreicher Nebenprodukte.

Aus DE-A1 Nr. 2708185 ist ferner ein Verfahren bekannt, wonach bestimmte aliphatische und cycloaliphatische Acylcyanide direkt zu den entsprechenden α-Ketocarbonsäureamiden bzw. den freien α-Ketocarbonsäuren hydrolysiert werden können. Dieses Verfahren ist jedoch, wie eigene Versuche gezeigt haben, auf die analoge Hydrolyse von Pivaloylcyanid nicht anwendbar, so dass der an sich attraktive Verfahrensweg von Pivaloylcyanid über Trimethylbrenztraubensäureamid und Trimethylbrenztraubensäure (gemäss DE-A1 Nr. 2708185) zum Triazinon I entfällt.

Es wurde nun gefunden, dass man 4-Amino-6-tert.-butyl-3-methylthio-1,2,4-triazin-5-[4H]-on der Formel I:

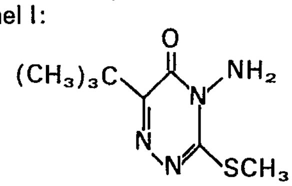

(I)

ausgehend von Pivaloylcyanid in überraschend einfacher Weise mit hoher Ausbeute und in hoher Reinheit erhält, wenn man in einer ersten Stufe Pivaloylcyanid der Formel II:

$$(CH_3)_3C-CO-CN \quad (II)$$

mit einem Carbonsäureanhydrid der allgemeinen Formel III:

$$R-CO-O-CO-R \quad (III)$$

in welcher

R für gegebenenfalls chlorsubstituiertes Alkyl mit 1 bis 4 Kohlenstoffatomen oder für Phenyl steht,

in Gegenwart einer starken Säure, ausgewählt aus der Gruppe bestehend aus anorganischen Sauerstoffsäuren, Lewis-Säuren, aliphatischen und aromatischen Sulfon- und Phosphonsäuren sowie Halogenalkancarbonsäuren, und gegebenenfalls in Gegenwart eines organischen Lösungsmittels bei Temperaturen zwischen −50 und 150°C umsetzt und das so erhaltene Reaktionsgemisch anschliessend direkt mit Thiocarbohydrazid der Formel IV:

$$NH_2-NH-CS-NH-NH_2 \quad (IV)$$

umsetzt und das hierbei gebildete 4-Amino-6-tert.-butyl-3-mercapto-1,2,4-triazin-5-[4H]-on der Formel V:

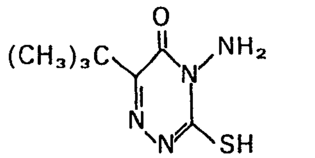

(V)

abtrennt und in einer zweiten Stufe dieses Zwischenprodukt V in üblicher Weise methyliert.

Die erste Stufe des erfindungsgemässen Verfahrens beinhaltet eine gegenüber dem Stand der Technik absolut neuartige und vorteilhafte Verfahrensweise, wobei es in überraschend glattem und einheitlichem Reaktionsverlauf erstmalig gelingt, Pivaloylcyanid unter milden Bedingungen in einem Eintopfverfahren, ohne dass irgendwelche Zwischenprodukte isoliert werden müssen, direkt mit nahezu quantitativer Ausbeute in ausserordentlich reines 4-Amino-6-tert.-butyl-3-mercapto-1,2,4-triazin-5-[4H]-on V zu überführen.

Das erfindungsgemässe Verfahren vermeidet die zuvor erwähnten, mit den vergleichbaren vorbekannten Verfahren zur Herstellung des herbiziden Wirkstoffes I verbundenen Nachteile; dies bedeutet eine sehr erhebliche technische Vereinfachung.

Gegenüber anderen vorbekannten Verfahren (vgl. z.B. DE-OS Nrn. 2003144, 2460889, 2460909, 2648300) zur Herstellung des Wirkstoffes I aus anderen Pivalinsäurederivaten bzw. Pinakolin hat das erfindungsgemässe Verfahren ebenfalls den technischen Vorteil starker Vereinfachung. Gegenüber den von Pinakolin ausgehenden Verfahren ist ein zusätzlicher Vorteil in der unterschiedlichen Rohstoffbasis zu sehen.

Verwendet man in der ersten Verfahrensstufe Essigsäureanhydrid als Carbonsäureanhydrid der allgemeinen Formel III und konzentrierte Schwefelsäure als starke Säure und verwendet man in der zweiten Stufe Methylbromid als Methylierungsmittel, so kann der Reaktionsverlauf nach dem erfindungsgemässen Verfahren zusammenfassend nochmals durch das folgende Formelschema wiedergegeben werden:

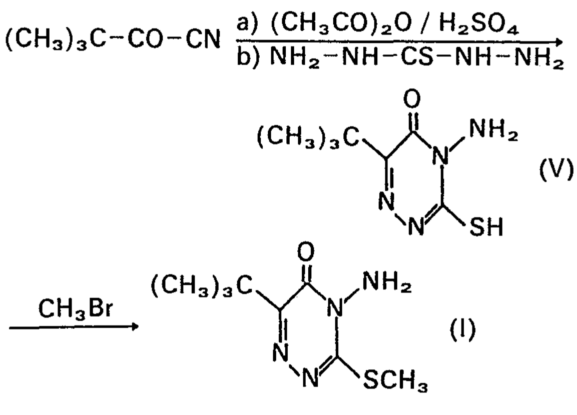


(V)

(I)

Das als Ausgangsprodukt verwendete Pivaloylcyanid II ist bekannt und kann z.B. durch Umsetzung von Pivaloylchlorid mit Kupfer(I)cyanid hergestellt werden (vgl. z.B. „J. Amer. Chem. Soc." *72*, S. 2793 [1950]).

Die weiterhin als Ausgangsstoffe einzusetzenden Carbonsäureanhydride der Formel III sind zum Teil grosstechnisch verfügbar bzw. nach allgemein bekannten Methoden z.B. aus den entsprechenden Carbonsäuren herstellbar.

Im Rahmen dieser Erfindung sind Essigsäureanhydrid, Propionsäureanhydrid und die Anhydride der Chloressigsäuren besonders bevorzugte Carbonsäureanhydride.

Zu Stufe 1 des erfindungsgemässen Verfahrens seien im einzelnen noch folgende Angaben gemacht:

Die erste Verfahrensstufe wird in Gegenwart einer starken Säure durchgeführt. Als solche Säuren kommen anorganische Sauerstoffsäuren, wie konzentrierte Schwefelsäure, sowie Perchlorsäure, Salpetersäure und Phosphorsäure, ferner Lewis-Säuren, wie Bortrifluorid, Aluminiumchlorid oder Zinkchlorid in Frage. Weiterhin geeignet sind aliphatische und aromatische Sulfon- und Phosphonsäuren sowie Halogenalkancarbonsäuren, wie z.B. Trichloressigsäure. Es ist auch möglich, die Umsetzung in Gegenwart mehrerer solcher Säuren durchzuführen. Bevorzugt werden anorganische Sauerstoffsäuren, insbesondere konzentrierte Schwefelsäure verwendet.

Die Reaktionstemperaturen können bei dieser Verfahrensstufe in einem grösseren Bereich variiert werden. Im allgemeinen arbeitet man, wie oben angegeben, bei Temperaturen zwischen etwa −50 und 150°C, vorzugsweise zwischen etwa 0 und 100°C.

Die Reaktion wird im allgemeinen bei Normaldruck durchgeführt.

Die Umsetzung gemäss Verfahrensstufe 1 kann in Abwesenheit oder in Gegenwart eines Lösungsmittels bzw. Lösungsvermittlers durchgeführt werden. Als Lösungsvermittler kommen bestimmte organische Lösungsmittel in Frage; besonders geeignet sind Eisessig und Dichlormethan, ferner Dialkyläther, wie Diäthyl- oder Diisopropyläther, und Diaryläther, wie z.B. Diphenyläther.

Bei der Durchführung der ersten Stufe des erfindungsgemässen Verfahrens setzt man im allgemeinen auf 1 mol Pivaloylcyanid der Formel II 0,5 bis 6 mol, vorzugsweise 0,8 bis 4 mol Carbonsäureanhydrid der Formel III ein; besonders bevorzugt ist ein Molverhältnis Pivaloylcyanid II zu Carbonsäureanhydrid III von 1:1 bis 1:2.

Die für die Durchführung der ersten Stufe des erfindungsgemässen Verfahrens erforderlichen Säuren werden in katalytischen bis überstöchiometrischen Mengen eingesetzt. Im allgemeinen setzt man auf 1 mol Pivaloylcyanid II 0,5 bis 10 mol, vorzugsweise 0,8 bis 8 mol, besonders bevorzugt 1 bis 4 mol Säure ein.

Besonders vorteilhaft ist ein Molverhältnis Carbonsäureanhydrid III zu der starken Säure von 1:2.

Ferner ist es besonders zweckmässig, Pivaloylcyanid II und Thiocarbohydrazid IV in äquimolaren Mengen einzusetzen.

Damit ergibt sich, dass es bei der Durchführung von Verfahrensstufe 1 besonders günstig ist, Pivaloylcyanid II, Carbonsäureanhydrid III, die starke Säure und Thiocarbonhydrazid IV im Molverhältnis von 1:1:2:1 bis 1:2:4:1 umzusetzen.

Wenn das Verfahren in technischem Massstab durchgeführt wird, kann es jedoch vorteilhaft sein, unter Konstanthalten des Molverhältnisses Carbonsäureanhydrid III zur starken Säure von 1:2 einen Überschuss dieser beiden Komponenten einzusetzen, um das anfallende Reaktionsgemisch gut rührbar zu halten.

Zweckmässigerweise geht man bei der Durchführung der ersten Stufe des erfindungsgemässen Verfahrens wie folgt vor:

Man legt die starke Säure bzw. ein Gemisch aus Lösungsmittel und der starken Säure vor und fügt nacheinander das Carbonsäureanhydrid III und das Pivaloylcyanid II zu; das so erhaltene Reaktionsgemisch wird entweder sofort nach beendeter Zugabe des Pivaloylcyanids oder nach einer gewissen Nachrührzeit (von max. 3 h) in eine vorzugsweise mineralsaure wässerige oder wässerigalkoholische Lösung oder in eine wässerige Suspension von Thiocarbohydrazid IV eingetragen. Es ist jedoch auch möglich, umgekehrt die Thiocarbohydrazidlösung bzw. -suspension in das vorerwähnte Reaktionsgemisch einzutragen.

Die Reaktionszeiten betragen im allgemeinen 1 bis 10 h. Das Reaktionsprodukt der ersten Verfahrensstufe fällt in der Regel in kristalliner Form aus und kann in üblicher Weise durch Filtration oder durch Extraktion isoliert werden.

Hierzu geeignete Extraktionsmittel sind mit Wasser nicht beliebig mischbare Lösungsmittel, beispielsweise Äther wie Diäthyläther oder Diisopropyläther, Ester wie z.B. Essigsäureäthylester, Ketone wie z.B. Methylisobutylketon, Halogenkohlenwasserstoffe wie z.B. Dichlormethan, Chlorbenzol oder Dichlorbenzol, ferner Aromaten wie z.B. Benzol, Toluol, o-Xylol, Äthylbenzol, Cumol oder Nitrobenzol. Vorzugsweise verwendet man Dichlormethan.

Das Zwischenprodukt V kann auch in Form des tautomeren 4-Amino-6-tert.-butyl-5-oxo-3-thioxotetrahydro-1,2,4-[2H,4H]-triazins der Formel:

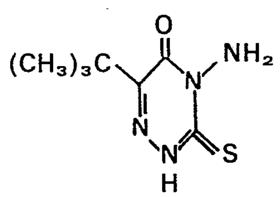

(Va)

vorliegen; der Einfachheit wegen wird hier jedoch für beide Tautomeren V bzw. Va stets die Bezeichnung 4-Amino-6-tert.-butyl-3-mercapto-1,2,4-triazin-5-[4H]-on V verwendet.

Die zweite Stufe des erfindungsgemässen Verfahrens erfolgt in bekannter Weise durch Umsetzung von V beispielsweise mit einem Methylhalogenid, wie Methylbromid oder Methyljodid, in Gegenwart einer Base, wie Natriumhydroxid, in wässeriger Lösung bei Temperaturen zwischen 0 und 50°C.

Das erfindungsgemäss herstellbare 4-Amino-6-tert.-butyl-3-methylthio-1,2,4-triazin-5-[4H]-on der Formel I (Metribuzin) zeichnet sich bekanntermassen durch hervorragende herbizide Wirksamkeit aus (vgl. z.B. DE-PS Nr. 1795784).

Die nachfolgenden Herstellungsbeispiele sollen das erfindungsgemässe Verfahren näher illustrieren.

*Beispiel 1:*

A) *Stufe 1:*

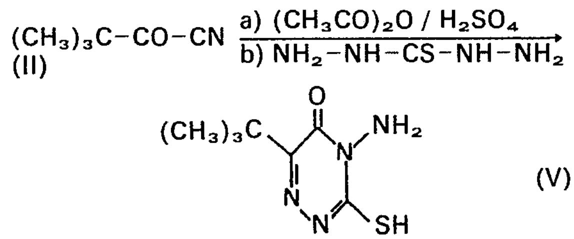


In 49,0 g (0,5 mol) vorgelegte konzentrierte Schwefelsäure werden jeweils bei Raumtemperatur zunächst 25,6 g (0,25 mol) Essigsäureanhydrid und anschliessend 27,8 g (0,25 mol) Pivaloylcyanid eingetragen. Nach ½stündigem Nachrühren wird dieses Reaktionsgemisch in eine Lösung von 26,6 g (0,25 mol) Thiocarbohydrazid in 300 ml 1N-HCl bei 20-30°C eingerührt. Nach beendeter Zugabe wird weitere 1,5 h bei 50-55°C nachgerührt; nach Abkühlen wird das ausgefallene Reaktionsprodukt abgesaugt, mit 200 ml Wasser gewaschen und getrocknet. Man erhält 48,6 g (97% d. Th.) 4-Amino-6-tert.-butyl-3-mercapto-1,2,4-triazin-5-[4H]-on V als farblose Kristalle vom Schmelzpunkt 212-214°C; Gehalt nach gaschromatographischer Bestimmung > 99%. Für anschliessende Umsetzungen sind keine weiteren Reinigungsoperationen erforderlich.

B) *Stufe 2:* Methylierung V → I

In eine Mischung aus 236 g 45%iger Natronlauge und 160 g Wasser werden unter Rühren 48,6 g (0,243 mol) 4-Amino-6-tert.-butyl-3-mercapto-1,2,4-triazin-5-[4H]-on V eingetragen. Nach vollständiger Lösung des Produktes werden 40,2 g Methyljodid so zugegeben, dass die Innentemperatur 30°C nicht übersteigt. Nach beendeter Zugabe wird das Reaktionsgemisch noch 2 h bei Raumtemperatur gerührt. Dann wird das ausgefallene Reaktionsprodukt abgesaugt, mit 100 ml Wasser gewaschen und getrocknet. Man erhält 42,5 g (82% d. Th.) 4-Amino-6-tert.-butyl-3-methylthio-1,2,4-triazin-5-[4H]-on I vom Schmelzpunkt 123-125°C.

*Beispiel 2:*

A) Es wird wie in Beispiel 1 A beschrieben verfahren, jedoch wird statt Essigsäureanhydrid die äquivalente Menge (0,25 mol) Propionsäureanhydrid eingesetzt. Man erhält 42,0 g (84% d. Th.) 4-Amino-6-tert.-butyl-3-mercapto-1,2,4-triazin-5-[4H]-on V.

B) Die Methylierung zu I erfolgt entsprechend Beispiel 1 B.

*Beispiel 3:*

A) Es wird wie in Beispiel 1 A beschrieben verfahren, jedoch wird die Reaktionsmischung aus konzentrierter Schwefelsäure, Essigsäureanhydrid und Pivaloylcyanid in eine wässerige Suspension aus 26,6 g (0,25 mol) Thiocarbohydrazid und 300 ml Wasser eingetragen. Man erhält 45,1 g (90,1% d. Th.) 4-Amino-6-tert.-butyl-3-mercapto-1,2,4-triazin-5-[4H]-on V vom Schmelzpunkt 212-214°C; Gehalt laut Gaschromatogramm > 99%.

B) Die Methylierung zu I erfolgt entsprechend Beispiel 1 B.

*Beispiel 4:*

A) *Stufe 1:* II → V/halbtechnischer Massstab

In 9,81 kg (100 mol) vorgelegte konzentrierte Schwefelsäure werden jeweils bei Raumtemperatur zunächst 5,12 kg (50 mol) Essigsäureanhydrid und anschliessend 2,78 kg (25 mol) Pivaloylcyanid eingetragen. Nach 2stündigem Nachrühren wird dieses Reaktionsgemisch in eine Lösung von 2,66 kg (25 mol) Thiocarbonhydrazid in 30 l 1N-HCl bei 20-30°C eingerührt. Nach beendeter Zugabe wird weitere 2 h bei 50-55°C nachgerührt; nach Abkühlen wird das ausgefallene Reaktionsprodukt abgesaugt, mit 20 l Wasser gewaschen und getrocknet. Man erhält 4,80 kg (96% d. Th.) 4-Amino-6-tert.-butyl-3-mercapto-1,2,4-triazin-5-[4H]-on V als farblose Kristalle vom Schmelzpunkt 212-214°C; Gehalt nach gaschromatographischer Bestimmung > 99%.

Der gegenüber Beispiel 1 — bei konstant gehaltenem Molverhältnis Essigsäureanhydrid zu konz. $H_2SO_4$ von 1:2 — eingesetzte Überschuss an Essigsäureanhydrid/konz. $H_2SO_4$ dient dazu, bei einem solchen grösseren Ansatz während der gesamten Reaktionszeit die gute Rührfähigkeit des Reaktionsgemisches zu gewährleisten; die erzielten Ausbeuten sind in beiden Fällen praktisch die gleichen.

B) Die Methylierung zu I kann wie in Beispiel 1 B beschrieben erfolgen. Jedoch stehen für die grosstechnische Durchführung der Methylierung gemäss Stufe 2 auch verschiedene verbesserte Verfahren zur Verfügung (vgl. z.B. DE-OS Nr. 2729761, ferner US-PS Nrn. 3890317, 3897429, 3905973, 4035364).

**Patentansprüche**

1. Verfahren zur Herstellung von 4-Amino-6-tert.-butyl-3-methylthio-1,2,4-triazin-5-[4H]-on der Formel (I):

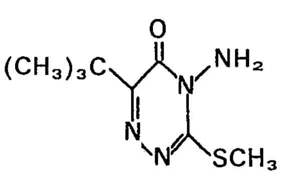

(I)

dadurch gekennzeichnet, dass man

(1) Pivaloylcyanid der Formel (II):

$$(CH_3)_3C-CO-CN \quad (II)$$

mit einem Carbonsäureanhydrid der allgemeinen Formel (III):

$$R-CO-O-CO-R \quad (III)$$

in welcher

R für gegebenenfalls chlorsubstituiertes Alkyl mit 1 bis 4 Kohlenstoffatomen oder für Phenyl steht,

in Gegenwart einer starken Säure, ausgewählt aus der Gruppe bestehend aus anorganischen Sauerstoffsäuren, Lewis-Säuren, aliphatischen und aromatischen Sulfon- und Phosphonsäuren sowie Halogenalkancarbonsäuren, und gegebenenfalls in Gegenwart eines organischen Lösungsmittels bei Temperaturen zwischen −50 und 150°C umsetzt und das so erhaltene Reaktionsgemisch anschliessend direkt mit Thiocarbohydrazid der Formel (IV):

$$NH_2-NH-CS-NH-NH_2 \quad (IV)$$

umsetzt und das hierbei gebildete 4-Amino-6-tert.-butyl-3-mercapto-1,2,4-triazin-5-[4H]-on der Formel (V):

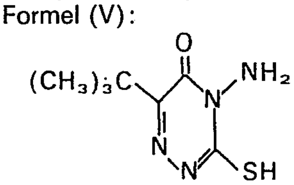

(V)

abtrennt, und

(2) dieses Zwischenprodukt (V)

in üblicher Weise methyliert.

2. Verfahren nach Anspruch 1, dadurch gekennzeichnet, dass man die Umsetzung gemäss Stufe (1) bei Temperaturen zwischen 0 und 100°C durchführt.

3. Verfahren nach Anspruch 1, dadurch gekennzeichnet, dass man bei der Umsetzung gemäss Stufe (1) Pivaloylcyanid (II) und Carbonsäureanhydrid (III) im Molverhältnis von 1:0,5-6 einsetzt.

4. Verfahren nach Anspruch 3, dadurch gekennzeichnet, dass man bei der Umsetzung gemäss Stufe (1) Pivaloylcyanid (II) und Carbonsäureanhydrid (III) im Molverhältnis von 1:0,8-4 einsetzt.

5. Verfahren nach Anspruch 4, dadurch gekennzeichnet, dass man bei der Umsetzung gemäss Stufe (1) Pivaloylcyanid (II) und Carbonsäureanhydrid (III) im Molverhältnis von 1:1-2 einsetzt.

6. Verfahren nach Anspruch 1, dadurch gekennzeichnet, dass man bei der Umsetzung gemäss Stufe (1) Pivaloylcyanid (II) und starke Säure im Molverhältnis von 1:0,5-10 einsetzt.

7. Verfahren nach Anspruch 6, dadurch gekennzeichnet, dass man bei der Umsetzung gemäss Stufe (1) Pivaloylcyanid (II) und starke Säure im Molverhältnis von 1:0,8-8 einsetzt.

8. Verfahren nach Anspruch 7, dadurch gekennzeichnet, dass man bei der Umsetzung gemäss Stufe (1) Pivaloylcyanid (II) und starke Säure im Molverhältnis von 1:1-4 einsetzt.

9. Verfahren nach Anspruch 1, dadurch gekennzeichnet, dass man bei der Umsetzung gemäss Stufe (1) Carbonsäureanhydrid (III) und starke Säure im Molverhältnis von 1:2 einsetzt.

10. Verfahren nach Anspruch 1, dadurch gekennzeichnet, dass man bei der Umsetzung gemäss Stufe (1) Pivaloylcyanid (II), Carbonsäureanhydrid (III), starke Säure und Thiocarbohydrazid (IV) im Molverhältnis von 1:1:2:1 bis 1:2:4:1 einsetzt.

11. Verfahren nach Anspruch 1, dadurch gekennzeichnet, dass man bei der Umsetzung gemäss Stufe (1) als Carbonsäureanhydrid der Formel (III) Essigsäureanhydrid einsetzt.

12. Verfahren nach Anspruch 1, dadurch gekennzeichnet, dass man bei der Umsetzung gemäss Stufe (1) als starke Säure konzentrierte Schwefelsäure einsetzt.

13. Verfahren zur Herstellung von 4-Amino-6-tert.-butyl-3-mercapto-1,2,4-triazin-5-[4H]-on (V) nach Stufe (1) des Verfahrens gemäss Anspruch 1.

## Revendications

1. Procédé de préparation de la 4-amino-6-tert.-butyl-3-méthylthio-1,2,4-triazin-5-[4H]-one de formule (I):

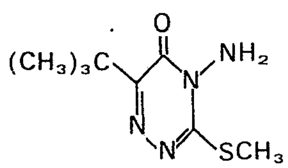

(I)

caractérisé en ce que

(1) on fait réagir du cyanure de pivaloyle de formule (II):

$$(CH_3)_3C-CO-CN \quad (II)$$

avec un anhydride d'acide carboxylique de formule générale (III):

$$R-CO-O-CO-R \quad (III)$$

dans laquelle:

R représente un groupe alkyle contenant 1 à 4 atomes de carbone et éventuellement substitué par un atome de chlore, ou un groupe phényle, en présence d'un acide fort choisi parmi le groupe comprenant les acides oxygénés inorganiques, les acides de Lewis, les acides sulfoniques et phosphoniques aliphatiques et aromatiques, de même que les acides halogénoalcanecarboxyliques, ainsi qu'éventuellement en présence d'un solvant organique à des températures comprises entre −50 et 150°C, et on fait ensuite réagir directement le mélange réactionnel ainsi obtenu avec le thiocarbohydrazide de formule (IV):

$$NH_2-NH-CS-NH-NH_2 \quad (IV)$$

et l'on sépare la 4-amino-6-tert.-butyl-3-mercapto-1,2,4-triazin-5-[4H]-one ainsi formée de formule (V):

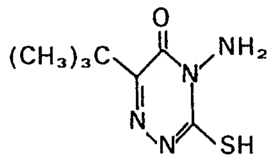

(V)

et

(2) on soumet ce produit intermédiaire (V) à une méthylation de la manière habituelle.

2. Procédé suivant la revendication 1, caractérisé en ce qu'on effectue la réaction suivant l'étape (1) à des températures comprises entre 0 et 100°C.

3. Procédé suivant la revendication 1, caractérisé en ce que, lors de la réaction suivant l'étape (1), on utilise le cyanure de pivaloyle (II) et l'anhydride d'acide carboxylique (III) dans un rapport molaire de 1:0,5-6.

4. Procédé suivant la revendication 3, caractérisé en ce que, lors de la réaction suivant l'étape (1), on utilise le cyanure de pivaloyle (II) et l'anhydride d'acide carboxylique (III) dans un rapport molaire de 1:0,8-4.

5. Procédé suivant la revendication 4, caractérisé en ce que, lors de la réaction suivant l'étape (1), on utilise le cyanure de pivaloyle (II) et l'anhydride d'acide carboxylique (III) dans un rapport molaire de 1:1-2.

6. Procédé suivant la revendication 1, caractérisé en ce que, lors de la réaction suivant l'étape (1), on utilise le cyanure de pivaloyle (II) et l'acide fort dans un rapport molaire de 1:0,5-10.

7. Procédé suivant la revendication 6, caractérisé en ce que, lors de la réaction suivant l'étape (1), on utilise le cyanure de pivaloyle (II) et l'acide fort dans un rapport molaire de 1:0,8-8.

8. Procédé suivant la revendication 7, caractérisé en ce que, lors de la réaction suivant l'étape (1), on utilise le cyanure de pivaloyle (II) et l'acide fort dans un rapport molaire de 1:1-4.

9. Procédé suivant la revendication 1, caractérisé en ce que, lors de la réaction suivant l'étape (1), on utilise l'anhydride d'acide carboxylique (III) et l'acide fort dans un rapport molaire de 1:2.

10. Procédé suivant la revendication 1, caractérisé en ce que, lors de la réaction suivant l'étape (1), on utilise le cyanure de pivaloyle (II), l'anhydride d'acide carboxylique (III), l'acide fort et le thiocarbohydrazide (IV) dans un rapport molaire de 1:1:2:1 à 1:2:4:1.

11. Procédé suivant la revendication 1, caractérisé en ce que, lors de la réaction suivant l'étape (1), comme anhydride d'acide carboxylique de formule (III), on utilise l'anhydride d'acide acétique.

12. Procédé suivant la revendication 1, caractérisé en ce que, lors de la réaction suivant l'étape (1), comme acide fort, on utilise l'acide sulfurique concentré.

13. Procédé de préparation de la 4-amino-6-tert.-butyl-3-mercapto-1,2,4-triazin-5-[4H]-one (V) suivant l'étape (1) du procédé selon la revendication 1.

## Claims

1. Process for the preparation of 4-amino-6-tert.-butyl-3-methylthio-1,2,4-triazin-5-[4H]-one of the formula (I):

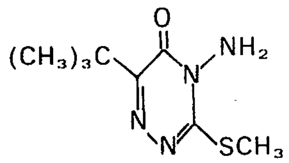

(I)

characterised in that
(1) pivaloyl cyanide of the formula (II):

$$(CH_3)_3C-CO-CN \quad (II)$$

is reacted with a carboxylic acid anhydride of the general formula (III):

$$R-CO-O-CO-R \quad (III)$$

in which
R represents optionally chlorine-substituted alkyl with 1 to 4 carbon atoms or phenyl,
in the presence of a strong acid selected from the group consisting of inorganic oxyacids, Lewis acids, aliphatic and aromatic sulphonic acids and phosphonic acids and halogenoalkanecarboxylic acids, and if appropriate in the presence of an organic solvent, at temperatures between −50 and 150°C and the reaction mixture thus obtained is then reacted directly with thiocarbohydrazide of the formula (IV):

$$NH_2-NH-CS-NH-NH_2 \quad (IV)$$

and the 4-amino-6-tert.-butyl-3-mercapto-1,2,4-triazin-5-[4H]-one formed in this reaction, of the formula (V):

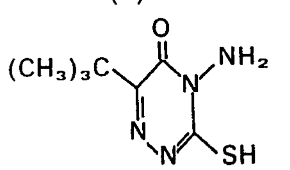

(V)

is separated off, and
(2) this intermediate product (V)
is methylated in the customary manner.

2. Process according to claim 1, characterised in that the reaction in stage (1) is carried out at temperatures between 0 and 100°C.

3. Process according to claim 1, characterised in that, in the reaction in stage (1), pivaloyl cyanide (II) and the carboxylic acid anhydride (III) are employed in a molar ratio of 1:0.5-6.

4. Process according to claim 3, characterised in that, in the reaction in stage (1), pivaloyl cyanide (II) and the carboxylic acid anhydride (III) are employed in a molar ratio of 1:0.8-4.

5. Process according to claim 4, characterised in that, in the reaction in stage (1), pivaloyl cyanide (II) and the carboxylic acid anhydride (III) are employed in a molar ratio of 1:1-2.

6. Process according to claim 1, characterised in that, in the reaction in stage (1), pivaloyl cyanide (II) and the strong acid are employed in a molar ratio of 1:0.5-10.

7. Process according to claim 6, characterised in that, in the reaction in stage (1), pivaloyl cyanide (II) and the strong acid are employed in a molar ratio of 1:0.8-8.

8. Process according to claim 7, characterised in that, in the reaction in stage (1), pivaloyl cyanide (II) and the strong acid are employed in a molar ratio of 1:1-4.

9. Process according to claim 1, characterised in that, in the reaction in stage (1), the carboxylic acid anhydride (III) and the strong acid are employed in a molar ratio of 1:2.

10. Process according to claim 1, characterised in that, in the reaction in stage (1), pivaloyl cyanide (II), the carboxylic acid anhydride (III), the strong acid and thiocarbohydrazide (IV) are employed in a molar ratio of 1:1:2:1 to 1:2:4:1.

11. Process according to claim 1, characterised in that, in the reaction in stage (1), acetic anhydride is employed as the carboxylic acid anhydride of the formula (III).

12. Process according to claim 1, characterised in that, in the reaction in stage (1), concentrated sulphuric acid is employed as the strong acid.

13. Process for the preparation of 4-amino-6-tert.-butyl-3-mercapto-1,2,4-triazin-5-[4H]-one (V) by stage (1) of the process according to claim 1.